# EUROPÄISCHE PATENTANMELDUNG

(11) **EP 0 767 368 A2**
(43) Veröffentlichungstag der Anmeldung: **09.04.1997**
(21) Anmeldenummer: 96114045.6
(22) Anmeldetag: 03.09.1996
(51) Int. Cl.: G01N 1/26

(54) **Verfahren und Vorrichtung zur Entnahme von Gasproben aus Behältern**

(30) Priorität: 06.10.1995 CH 2824/95
(71) Anmelder: ELPATRONIC AG, CH-6303 Zug (CH)
(72) Erfinder: Rosatzin, Martin, Dr., 8953 Dietikon (CH)

(57) **Zusammenfassung**

Zur Entnahme einer Gasprobe aus einem Behälter (1) wird ein Gasstrom (3) in den Behälter eingeleitet und dadurch das im Behälter befindliche Gas (5) aus diesem verdrängt. Das verdrängte Gas (5) wird in einen Sammelraum (4) eingeleitet, dessen Eintrittsöffnung grösser ist als dessen Austrittsöffnung. Oberhalb der Austrittsöffnung wird durch eine Saugleitung (7) ein Teil (8) des aus der Flasche ausgestossenen Gasstromes abgesaugt und zur Analyse einer Analysestation zugeführt. Durch dieses Verfahren bzw. diese Anordnung ergibt sich eine Ausnutzung des verdrängten Gasstromes über einen längeren Zeitraum ohne namhafte Verdünnung und damit eine verbesserte Analysemöglichkeit.

## Beschreibung

Die Erfindung betrifft ein Verfahren zur Entnahme einer Gasprobe aus Behältern, die mittels einer Fördereinrichtung an einer Entnahmestation vorbeigeführt werden, in welcher in die Behälter jeweils ein Medium eingeblasen wird und das dadurch aus dem Behälter verdrängte Gas mindestens teilweise in einen durchströmbaren Sammelraum aufgenommen und zu mindestens einer Analyseeinrichtung abgesaugt wird. Ferner betrifft die Erfindung eine Vorrichtung zur Durchführung des Verfahrens gemäss Oberbegriff des Anspruchs 9.

Aus DE-C-42 25 984 ist ein Verfahren gemäss Oberbegriff bzw. eine solche Vorrichtung bekannt. Dadurch soll auf einfache Weise eine Gasprobe aus Behältern, insbesondere aus rücklaufenden Mehrweg-Flaschen, entnehmbar sein. Bei dem Verfahren bzw. der Vorrichtung gemäss Stand der Technik ist die dort angestrebte und erreichte mechanische Vereinfachung indes mit einer sehr kurzen Gasentnahmezeit verbunden, was insbesonders bei einer raschen Flaschenförderung ungenügende Resultate bringen kann. Aus WO-A-93/24841 ist ein Verfahren bzw. eine Vorrichtung bekannt, bei dem grundsätzlich die selben Probleme auftreten.

Die nachveröffentlichte Schrift DE-A-44 27 314 zeigt eine Anordnung, bei der keine Absaugung aus einem Sammelraum erfolgt, sondern bei der das ausgestossene Gas direkt durch einen Kanal einer Messküvette zugeleitet und dann in die Umgebung entlassen wird.

Der vorliegenden Erfindung liegt daher die Aufgabe zugrunde, das bekannte Verfahren zu verbessern, so dass eine Entnahme analysierfähiger Gasproben sicher auch unter erschwerten Bedingungen, z.B. bei hoher Flaschenkadenz, durchführbar ist.

Diese Aufgabe wird bei dem eingangs genannten Verfahren dadurch gelöst, dass der Eintritt des Gases in den Sammelraum über einen längeren Förderwegabschnitt erfolgt als der Austritt des Gases aus dein Sammelraum.

Es hat sich gezeigt, dass durch ein solches Vorgehen, bei dem das aus dem Behälter ausgetriebene Gas über einen längeren Bereich des Förderweges gesammelt und auf ein kleineres Raumvolumen im Bereich der Austrittsöffnung fokussiert wird, das genannte Problem lösbar ist. Für jeden Behälter steht die über einen Förderwegabschnitt - und nicht nur die an einer bestimmten Förderwegstelle, wie im Stand der Technik - aus dem Behälter durch die Einblasluft verdrängte Gasprobe zur Verfügung.

Vorteilhafterweise erfolgt weiter die Absaugung der Gasprobe oberhalb der Austrittsöffnung des Sammelraumes. Damit wird die Strömung des aufgefangenen Gases im Sammelraum nicht durch das Absaugelement gestört und das Absaugelement befindet sich optimal im Gasstrom, was die Absaugung einer analysierfähigen Gasprobe begünstigt.

Weiter vorteilhaft ist es, wenn die Einblasung des Mediums, welches in der Regel Druckluft ist, ebenfalls über einen Förderwegabschnitt erfolgt, wobei dieser gleich lang oder länger als die Länge der Eintrittsöffnung des Sammelraumes sein kann. Eine kürzere Einblasung ist ebenfalls möglich, wird aber nicht bevorzugt.

Die Vorrichtung zur Durchführung des Verfahrens weist die kennzeichnenden Merkmale des Anspruchs 9 auf, wodurch die vorstehend genannte Aufgabe gelöst wird.

Die Erfindung wird nachstehend anhand der in den Zeichnungen als Beispiel dargestellten Ausführungsformen näher erläutert. Dabei zeigt
Figur 1 schematisch den Hals- und Mündungsbereich einer Flasche zur Erläuterung des Verfahrens;
Figur 2a schematisch den Sammelraum gemäss der Erfindung in Seitenansicht,
Figur 2b schematisch die Anordnung gemäss Figur 2a in Förderrichtung,
Figur 3 eine perspektivische Ansicht des Sammelraums mit der Einblasdüse,
Figur 4 eine schematische Darstellung zur Erläuterung der Spülluftführung,
Figur 5 ein Diagramm zur Erläuterung der Wirkung der Spülluft, und
Figur 6 eine Ansicht des Mündungsbereichs eines Behälters zur Erläuterung einer weiteren Luft- und Gasführung.

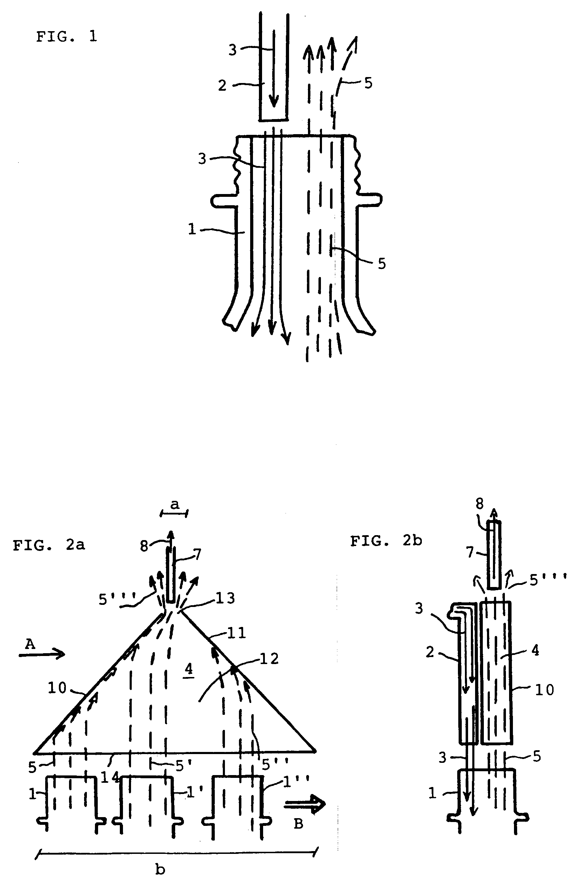

Figur 1 zeigt schematisch den oberen Teil eines Behälters 1, der z.B. eine Flasche ist. Es ist aus dem Stand der Technik bekannt, in diesen Behälter mittels einer Düse 2 einen Strahl 3 eines Mediums einzublasen. Das Medium wird in der Regel Luft sein, es kommt indes auch ein anderes gasförmiges Medium in Frage. Das Medium wird über einen Teil des Oeffnungsquerschnittes des Behälters 1 eingeblasen. Es ist weiter bekannt, dass durch das Einblasen des Mediums, das in der Flasche befindliche Gas durch Verdrängung durch das eingeblasene Medium aus der Flasche ausgetrieben wird. Dabei erfolgt das Austreiben während einer gewissen Zeit praktisch ohne Verdünnung durch das eingeblasene Medium. In Figur 1 ist das ausgeblasene Gas als Strömung 5 dargestellt. Es ist weiter bekannt, diese Strömung 5 in einem runden Sammelrohr aufzufangen und aus dem Inneren des Sammelrohrs einen Teil dieser Gasströmung abzusaugen, damit das Gas auf Rückstände analysiert werden kann, die einen Schluss auf die Wiederverwendbarkeit des Behälters zulassen. Die Behälter 1 werden auf einer Fördereinrichtung gefördert und passieren eine Entnahmestation, wo punktuell der Gasstrom 5 in das Sammelrohr eintritt.

Gemäss der Erfindung erfolgt nun der Eintritt des aus der Flasche 1 verdrängten Gases nicht mehr punktuell an einer Stelle des Förderweges, sondern über einen längeren Förderwegabschnitt, der länger ist als die Austrittsöffnung für das Gas oben am Sammelraum. In Figur 2a und 2b ist dies schematisch dargestellt, wobei in Figur 2a eine Seitenansicht des Sammelraums dargestellt ist, und die Flaschen in Richtung des Pfeiles B durch eine bekannte, nicht dargestellte Fördereinrichtung gefördert werden, während in Figur 2b derselbe Sammelraum 4 in Richtung des Pfeiles A in Figur 2a betrachtet wird, also in der Richtung der Behälterförderung. In Figur 2a ist ein Behälter 1 in drei verschiedenen Stellungen dargestellt, die er während der Förderung im Bereich des Sammelraumes einnimmt. Die drei dargestellten Stellungen sind dabei mit 1, 1' und 1'' bezeichnet. Entsprechend sind die in diesen Stellungen aus dem Behälter austretenden Gasströmungen mit 5, 5' und 5'' bezeichnet. In Figur 2a ist das in die Flaschen eingeblasene Medium nicht dargestellt. Dies wird aber aus der Figur 2b ersichtlich, was nachfolgend erläutert wird. In Figur 2a ist nun ersichtlich, dass der Sammelraum 4 eine Eintrittsöffnung 14 für die Gasströme 5 aufweist, welche sich über einen grösseren Förderwegabschnitt erstreckt als die Austrittsöffnung 13 für die aus dem Sammelraum austretenden Gasströme, welche mit 5''' bezeichnet sind. Im gezeigten Beispiel wird der Sammelraum 4 durch zwei z.B. als Leitbleche 10 und 11 ausgeführte Leitorgane oder Sammelorgane begrenzt, welche an ihrem unteren Ende weiter voneinander entfernt sind als an ihrem oberen Ende und mit ihren Enden so die Eintrittsöffnung 14 bzw. die Austrittsöffnung 13 definieren. Der Sammelraum kann an einer Seite mit einer Wand 12 abgeschlossen sein, oder er kann an beiden Seiten mit einer Wand abgeschlossen sein, oder er kann beidseitig offen sein. Es ist ersichtlich, dass durch die erfindungsgemässe Ausgestaltung des Sammelraumes während einer Förderstrecke b das aus dem jeweiligen Behälter 1 austretende Gas gesammelt wird. Es hat sich gezeigt, dass während hinreichend genügend langer Zeit ein im wesentlichen durch das eingeblasene Medium unverdünnter Gasstrom 5 aus der Flasche austritt. Durch die beanspruchte Eigenschaft des Sammelraums 4 ergibt sich eine Fokussierung der aus der Flasche austretenden Gasströme 5, 5' und 5'' bei der Austrittsöffnung 13 des Sammelraums. Der dort austretende Gasstrom ist als 5''' bezeichnet. Es hat sich weiter gezeigt, dass durch die Formgebung des Sammelraumes 4 eine nur geringe Verdünnung des Gasstromes 5 mit Umgebungsluft eintritt, da im wesentlichen nur bewegte Luft aus dem Behälter durch den Sammelraum 4 auf die Austrittsöffnung 13 geleitet wird. Vorzugsweise wird der der Analyse zuzuführende Teil des Gasstromes oberhalb der Austrittsöffnung 13 abgesaugt. Dazu ist ein Saugrohr 7 vorgesehen, welches einen Teil des Gasstromes 5''' absaugt und als Strom 8 einer nicht dargestellten Analyseeinheit zuführt. Die Anordnung der Ansaugung des Probegasstroms 8 oberhalb des Sammelraumes 4 hat den Vorteil, dass die Strömung im Sammelraum selber nicht gestört wird, so dass durch die Vermeidung einer solchen Störung dies Einbringung von Umgebungsluft weiter vermieden wird. Die Probegasströme 5, 5' und 5'' werden bei der Austrittsöffnung 13 des Sammelraums 4, die im Vergleich mit der Strecke b einen kleineren Austrittsdurchmesser a aufweist, fokussiert, so dass die oberhalb der Oeffnung 13 entnommene Gasprobe 8 eine repräsentative, nur wenig verdünnte Gasprobe aus dem Behälter 1 darstellt und zur Analyse während einer ungefähr um das Verhältnis b:a verlängerten Zeitspanne zur Verfügung steht.

Die in Figur 2a nicht dargestellte Einblasung des Druckluftstrahles 3 zur Verdrängung des im Behälter befindlichen Gases ist aus Figur 2b näher ersichtlich. Die Düse 2 zur Einbringung des Druckluftstrahles 3 befindet sich demgemäss in der Blickrichtung der Figur 2a hinter dem Sammelraum 4. Vorzugsweise ist die Düse 2 eine Düse, die sich über die gesamte Länge b der Eintrittsöffnung des Sammelraums 4 erstreckt. Die Düse 2 kann aber auch länger sein als die Strecke b, um bereits mit der Einblasung zu beginnen, bevor die Flaschenmündung den Bereich des Sammelraumes 4 erreicht. Auch eine kürzere Ausgestaltung der Düse 2 als die Strecke b ist möglich, wenn sich dies für eine bestimmte Anwendung als vorteilhaft erweist. In der Darstellung von Figur 2b bezeichnen dieselben Bezugszeichen, wie in 2a verwendet, dieselben Teile.

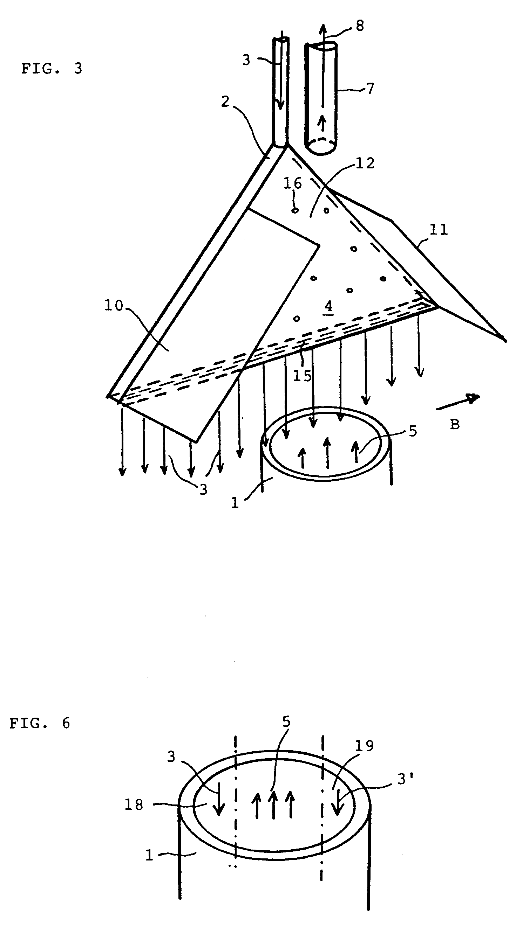

Figur 3 zeigt eine den Figuren 2a und 2b ähnliche Ausgestaltung der Vorrichtung zur Durchführung des Verfahrens in perspektivischer Darstellung. Dabei ist wieder die Mündung eines Behälters 1 dargestellt, welcher in Richtung des Pfeils B gefördert wird. Oberhalb des Behälters 1 ist der Sammelraum 4 angeordnet, welcher auch hier von zwei Leitblechen 10 und 11 begrenzt wird und ferner eine Rückwand 12 aufweist. Die Rückwand 12 ist ihrerseits Teil der Luftdüse 2, welche als Schlitzdüse mit einer schlitzförmigen Austrittsöffnung 15 ausgeführt ist, so dass sich eine Strömung 3 in Form eines Luftvorhanges ergibt, der während der ganzen Förderzeit des Behälters 1 unterhalb des Sammelraums 4 in den Behälter eintritt. Das aus dem Behälter 1 austretende Gas 5 ist in der Figur 3 der Klarheit halber nur an der Behältermündung dargestellt und nicht im Sammelraum. Die Gasströmung verläuft aber im wesentlichen so, wie in Figur 2a dargestellt. Oberhalb der Austrittsöffnung des Sammelraums 4 befindet sich das Absaugelement 7, welches aus dem Gasstrom 5 einen Teil als Gasstrom 8 entnimmt und der Analysestation zuführt. Im gezeigten Beispiel sind ferner Luftaustrittsöffnungen 16 in der Seitenwand 12 dargestellt, deren Funktion nachfolgend noch erläutert wird. Das Ausführungsbeispiel gemäss Figur 3 kann auf vielfältige Weise modifiziert werden. So können z.B. die Leitbleche 10 und 11 eine andere als die dargestellte rechteckige Form aufweisen. Ferner können diese Leitbleche anstatt gerade auch gekrümmt verlaufen, wobei die Krümmung sowohl eine Ausbuchtung zur Behältermündung hin als auch von dieser weg sein kann. Wie bereits erwähnt, ist weiter auch die Rückwand 12 des Sammelraumes nicht obligatorisch. Der Sammelraum könnte also gegen hinten ebenfalls offen sein. In diesem Fall würde die Luftzuführung zur Düse 2 nahe bei deren Austrittsöffnung 15 erfolgen und nicht oberhalb der Leitbleche 10 und 11. Diese wären bei Wegfall der Rückwand 12 auf andere Weise, z.B. durch stabförmige Halterungen, oberhalb der Fördereinrichtung befestigt. Die Menge und/oder der Druck des Mediums 3 wirdd bei allen Ausführungsformen entweder konstant eingestellt oder in Abhängigkeit von Betriebsparametern, wie z.B. der Fördergeschwindigkeit verändert.

Die gesamte den Sammelraum und die Absaugung bildende Vorrichtung ist ferner vorzugsweise höheneinstellbar angeordnet, so dass die Höhe der Entnahmestation über der Fördereinrichtung eingestellt werden kann, um die Vorrichtung an verschiedene Behälter anzupassen. Die schlitzförmige Oeffnung 15 der Düse 2 kann durch einzelne Oeffnungen ersetzt sein. In diesem Fall ergeben sich anstelle des bevorzugten kontinuierlichen Einblasens aus einem zusammenhängenden Luftvorhang mehrere nebeneinanderliegende kontinuierlich strömende Luftsäulen, die sich für die vorbeilaufende Flasche wie ein gepulstes Einblasen auswirken. Es ist ferner so, dass nicht nur eine Düse zur Einblasung in einem Kreisabschnitt des Mündungsbereiches vorgesehen sein kann, sondern auch zwei Düsen, die in zwei Kreisabschnitte einblasen Figur 6 zeigt schematisch eine solche Einblasung, wobei die eine Düse einen Luftvorhang 3 erzeugt, der in den Kreisabschnitt 18 einbläst, wie das auch bei Figur 3 der Fall ist, wobei aber gemäss Figur 6 eine weitere Düse vorgesehen ist, welche einen Luftvorhang 3' in einen Kreisabschnitt 19 der Mündung des Behälters 1 einbläst. Der austretende Gasstrom 5 verläuft dann zentral zwischen den beiden Luftvorhängen 3 und 3'. Eine entsprechende Abwandlung z.B. der Ausführungsform, wie sie in Figur 3 gezeigt ist, ist ohne weiteres möglich. Auch eine andere Variante ist natürlich möglich, und zwar dass die Einblasung zentral erfolgt, durch eine Düse, welche oberhalb des Kreismittelpunkts der Behälteröffnung verläuft und dass das seitlich in Kreisringabschnitten austretende Gas 5 in den Sammelraum gelangt. Für diesen Fall könnten allenfalls auch zwei separate Sammelräume vorgesehen sein, welche sich im Bereich ihrer Austrittsöffnung vereinigen. Die gesamte Gasentnahmeeinheit kann ferner beheizt sein, um eine Kondensation von Gas aus den Behältern an der Einheit auszuschliessen.

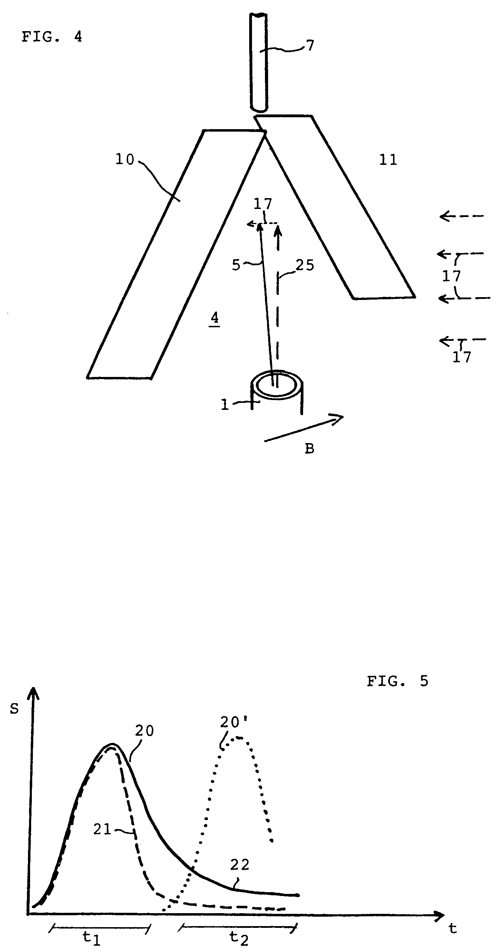

Anhand der Figur 4 wird nun eine weitere Ausführungsform erläutert. In der Figur 4 ist wiederum schematisch der Sammelraum 4 dargestellt, der durch die Leitbleche 10 und 11 begrenzt wird und an dessen Austrittsöffnung das Absaugrohr 7 angeordnet ist. Das aus dem Behälter 1 austretende Gas ist hier mit einem gestrichelten Pfeil 25 dargestellt, der die normale Austrittsrichtung des Gases darstellt. Bei der Ausführungsform gemäss Figur 4 wird nun eine Spülluftströmung 17 erzeugt, welche ungefähr quer zur Förderrichtung der Behälter verläuft und eine geringfügige Ablenkung des aus der Flasche 1 austretenden Gasstromes bewirkt. Der sich somit aufgrund der Querströmung 17 tatsächlich einstellende Gasstrom ist mit dem ausgezogenen Pfeil 5 bezeichnet. Auch dieser Gasstrom gelangt zur Absaugleitung 7, d.h. die Strömung 17 ist nur so schwach gewählt, dass der Gasstrom 5 weiterhin im Sammelraum verbleibt. Der Zweck dieser Spülluftströmung 17 ist es, den Gassammelraum 4 für den Eintritt des nachfolgenden Behälters 1 soweit zu spülen, dass sich, wenn dieser nachfolgende Behälter 1 seinen Gasstrom in den Sammelraum abgibt, nur noch geringfügige Mengen des aus dem vorgängigen Behälter ausgetretenen Gasstromes im Sammelraum befinden, damit die Analyse nicht durch diesen sogenannten Gedächtniseffekt des Sammelraumes gestört wird.

Figur 5 zeigt als Beispiel das Ausgangssignals einer Analysestation, welche den abgesaugten Gasstrom analysiert, über der Zeit t. Die Kurve 20 stellt dabei das Ausgangssignal dar, welches sich bei der Analyse des Gasstroms eines ersten Behälters ergeben hat, das Ausgangssignal 20', dasjenige Signal, welches sich bei der Analyse des nachfolgenden Behälters ergeben hat. Die Kurve 20 zeigt nun den Signalverlauf, wie er sich ohne den Einsatz von Spülluft ergibt. Es ist ersichtlich, dass im Zeitraum t2, wo der nachfolgende Behälter analysiert wird und das Ausgangssignal 20' aufgrund des austretenden Gasstromes erzeugt wird, noch Signalanteile 22 ermittelt werden, welche vom Gasstrom des vorgängig bei t1 analysierten Behälters herrühren. Mit dem Einsatz der Spülluft, ergibt sich anstelle der Kurve 20 die Kurve 21, bei der ersichtlich ist, dass die Gasanteile des vorgängigen Behälters im Zeitraum t2 im wesentlichen weggespült worden sind, so dass sich keine unzulässige Beeinflussung der Analyse des zweiten Behälterinhaltes ergibt.

Die Spülluftströmung 17 kann durch ein separates Gebläse oder einen separaten Druckluftanschluss erzeugt werden. Die Geschwindigkeit und Menge der Spülluftströmung wird dabei vorzugsweise an die jeweilige Behälterfördergeschwindigkeit und/oder Behälterart angepasst. In Figur 3 sind die bereits erwähnten Luftaustrittsöffnungen 16 in der Rückwand 12 des Sammelraums dargestellt, welche in diesem Beispiel zur Erzeugung des querverlaufenden Spülluftstromes verwendet werden. Der Spülluftstrom wird in diesem Beispiel direkt aus dem in die Flasche einzublasenden Luftstrom 3 abgenommen und in den Sammelraum geleitet.

## Patentansprüche

1. Verfahren zur Entnahme einer Gasprobe aus Behältern (1), die mittels einer Fördereinrichtung an einer Entnahmestation vorbeigeführt werden, in welcher in die Behälter jeweils ein Medium (3) eingeblasen wird und das dadurch aus dem Behälter verdrängte Gas (5) mindestens teilweise in einen durchströmbaren Sammelraum (4) aufgenommen und zu mindestens einer Analyseeinrichtung abgesaugt wird, dadurch gekennzeichnet, dass der Eintritt des Gases in den Sammelraum über einen längeren Förderwegabschnitt erfolgt als der Austritt des Gases (5''') aus dem Sammelraum.

2. Verfahren nach Anspruch 1, dadurch gekennzeichnet, dass der Eintritt über eine längliche, im wesentlichen parallel zur Fördereinrichtung verlaufende Eintrittsöffnung (14) des Sammelraumes (4) erfolgt.

3. Verfahren nach Anspruch 1 oder 2, dadurch gekennzeichnet, dass das Gas von oberhalb der Austrittsöffnung (13) des Sammelraumes abgesaugt wird.

4. Verfahren nach einem der Ansprüche 1 bis 3, dadurch gekennzeichnet, dass das Medium im wesentlichen über dieselbe oder über längere Zeit eingeblasen wird, als die Behälteröffnung der Eintrittsöffnung des Sammelraumes zugewandt ist.

5. Verfahren nach einem der Ansprüche 1 bis 4, dadurch gekennzeichnet, dass das Medium in der Form eines Luftvorhanges parallel zur Behälterförderrichtung eingeblasen wird.

6. Verfahren nach einem der Ansprüche 1 bis 5, dadurch gekennzeichnet, dass das Medium kontinuierlich oder pulsweise eingeblasen wird.

7. Verfahren nach einem der Ansprüche 1 bis 6, dadurch gekennzeichnet, dass die Menge und/oder der Druck des Mediums konstant eingestellt ist oder in Abhängigkeit mindestens eines Betriebsparameters, insbesonders der Behälterart, der Behältergrösse, der Behältertransportgeschwindigkeit, verändert wird.

8. Verfahren nach einem der Ansprüche 1 bis 7, dadurch gekennzeichnet, dass in den Sammelraum eine Spülströmung (17) im wesentlichen quer zur Förderrichtung eingebracht wird.

9. Vorrichtung zur Entnahme einer Gasprobe aus Behältern (1), umfassend eine Fördereinrichtung für die Behälter und eine Entnahmestation, wobei die Behälter mittels der Fördereinrichtung an der Entnahmestation vorbeiführbar sind, in welcher mittels einer Einblasanordnung ein Medium (3) in den jeweiligen Behälter (1) einblasbar und das dadurch aus dem Behälter verdrängte Gas in einem durchströmbaren Sammelraum (4) aufnehmbar ist, und umfassend eine Ansaugleitung (7), mittels derer ein Teil (8) des Gases einer Analyseeinrichtung zuführbar ist, dadurch gekennzeichnet, dass die der Fördereinrichtung zugewandte Eintrittsöffnung (14) des Sammelraumes grösser ist als die Austrittsöffnung (13).

10. Vorrichtung nach Anspruch 9, dadurch gekennzeichnet, dass die Eintrittsöffnung (14) als längliche, im wesentlichen parallel zur Fördereinrichtung verlaufende, Oeffnung ausgestaltet ist.

11. Vorrichtung nach Anspruch 9 oder 10, dadurch gekennzeichnet, dass die Ansaugleitung (7) oberhalb der Austrittsöffnung (13) des Sammelraumes angeordnet ist.

12. Vorrichtung nach einem der Ansprüche 9 bis 11, dadurch gekennzeichnet, dass die Einblasanordnung mindestens eine Schlitzdüse (2, 15) umfasst.

13. Vorrichtung nach Anspruch 12, dadurch gekennzeichnet, dass die Schlitzdüse seitlich neben der Eintrittsöffnung (14) des Sammelraumes angeordnet ist und im wesentlichen dieselbe Länge wie diese oder eine grössere Länge als diese aufweist.

14. Vorrichtung nach einem der Ansprüche 9 bis 13, dadurch gekennzeichnet, dass der Sammelraum durch zwei einander gegenüberliegende Leitbleche (10, 11) gebildet wird, die Teil einer Pyramidenstumpfmantelfläche sind.

15. Vorrichtung nach Anspruch 14, dadurch gekennzeichnet, dass der Sammelraum weiter mindestens eine die Leitbleche (10, 11) verbindende Seitenwand (12) aufweist.

16. Vorrichtung nach einem der Ansprüche 9 bis 15, dadurch gekennzeichnet, dass eine Einrichtung zur Erzeugung einer im wesentlichen quer zur Förderrichtung verlaufenden Spülströmung (17) im Sammelraum (4) vorgesehen ist.
